# EUROPEAN PATENT APPLICATION

(11) **EP 4 332 577 A1**
(43) Date of publication of application: **06.03.2024**
(21) Application number: 21939076.2
(22) Date of filing: 16.12.2021
(51) Int. Cl.: G01N 33/68

(54) **APPLICATIONS OF CRMP2 AND ANTI-CRMP2 ANTIBODIES**

(30) Priority: 27.04.2021 CN 202110461611
(71) Applicant: Nanfang Hospital, Southern Medical University, Guangzhou, Guangdong 510515 (CN)
(72) Inventor: PAN, Suyue, Guangzhou, Guangdong 510515 (CN); HU, Yafang, Guangzhou, Guangdong 510515 (CN); LIU, Guanghui, Guangzhou, Guangdong 510515 (CN); WANG, Shengnan, Guangzhou, Guangdong 510515 (CN); WANG, Dongmei, Guangzhou, Guangdong 510515 (CN); XU, Kaibiao, Guangzhou, Guangdong 510515 (CN); WU, Yongming, Guangzhou, Guangdong 510515 (CN)
(74) Representative: Bals & Vogel Patentanwälte PartGmbB
(86) International application number: PCT/CN2021/138864
(87) International publication number: WO 2022/227617

(57) **Abstract**

An application of CRMP2 and/or anti-CRMP2 antibodies as markers of a nervous system autoimmune disease, and an application of a reagent for detecting anti-CRMP2 antibodies in preparing a reagent for the detection, diagnosis, treatment or prognosis evaluation of a nervous system autoimmune disease. CRMP2 is a new encephalitis/encephalomyelitis-related autoantibody targeted molecular marker, which can be used for clear diagnosis of the disease and provide the basis for an immunotherapy solution. In addition, the treatment effect can be monitored and the prognosis and recurrence can be determined by detecting a titer change in CRMP2 autoantibodies.

## Description

### TECHNICAL FIELD

The present disclosure relates to the technical field of biomedicine, in particular to the application of CRMP2 and anti-CRMP2 antibodies.

### BACKGROUND

Autoimmune diseases of nervous system are a type of diseases caused by inflammation and other injuries due to autoimmune molecules and immune cells recognizing the cell components (autoantigens) of tissues of the nervous system, which can occur in central nervous system, peripheral nervous system and neuromuscular junction, and includes autoimmune encephalitis (AE), central demyelinating disease, autoimmune peripheral neuropathy, etc. Of these, AE is the most common. The clinical manifestations of acute or subacute onset (< 3 months), with one or more neurological and psychiatric symptoms or clinical syndromes: 1) limbic system symptoms: recent amnesia, epileptic seizure, and abnormal mental behaviors; 2) encephalitis syndrome: clinical manifestations of diffuse or multifocal brain damage; 3) clinical manifestations of basal ganglia and (or) diencephalon/hypothalamus involvement; 4) mental disorders that, in the opinion of the psychological specialists, do not correspond to a non-organic disorder. AE mainly affects young and middle-aged people, with high mortality and disability rates.

Currently, nearly 20 types of neuronal autoantigens have been discovered. The autoantigens discovered since 1985 were mainly directed against neuronal intracellular antibodies, such as anti-Hu (ANNA-1), Yo (PCA-1), Rui (ANNA 2), PNMA2 (paraneoplastic antigen Ma2). In 2019, Kelch-like Protein 11, a seminoma-associated paraneoplastic antigen, was newly discovered. Such antigens mainly mediate irreversible damage of neurons by cellular immunity, and the patients with the AE are often accompanied by tumors, also known as tumor associated AE or paraneoplastic syndrome. In 2007, an autoantibody against neuronal surface receptor AE, namely anti-NMDAR (N-methyl-D-aspartate receptor) antibody, was discovered for the first time in serum and cerebrospinal fluid (CSF) samples from teratoma patients in Dalmau Laboratory, Pennsylvania, USA. The pathological mechanism is that autoantibodies mediate humoral immune responses by binding to NMDAR receptors, which leads to a decrease in synaptic NMDAR receptors, thereby causing relatively reversible neuronal dysfunction with favorable immunotherapy effects. In recent years, an increasing number of humoral immunity mediated by autoantibodies targeting neuronal cell surface proteins and synaptic proteins have led to the identification of a new type of AE, and early immunotherapy for such AEs after diagnosis has been shown to be effective. Autoantigens (or proteins) corresponding to such AEs include: (1) neurotransmitter receptor AMPAR (alpha-amino-3-hydroxy-5-methyl-4-isoxazolepropionic acid receptor), mGluR5 (Metabotropic glutamate receptor 5), GABA_{A}R/GABA_{B}R (Gamma-Aminobutyric Acid A/B receptor), GlyR1 (Glycine receptor), D2R (Dopamine-2 receptor); (2) transmembrane protein CASPR2 (Contactin-associated protein-like 2), DNER (Delta/Notch Like EGF Repeat Containing), DDP6/DPPX (Dipetidyl Peptidase Like 6), DCC (Netrin 1 Receptor DCC), IgLON5 (IgLON Family Member 5), Neurexin-3α; (3) secreted protein LGI1 (Leucine Rich Glioma Inactivated 1); and (4) intracellular protein GAD65 (Glutamic Acid Decarboxylase 65). Unlike traditional paraneoplastic syndromes, AE may or may not be accompanied by tumorigenesis. Tumors and viral infections are known to be one of the inducing factors.

An expert consensus on the clinical diagnosis and treatment of AE was established by Graus et al. in 2016. In 2017, Chinese encephalitis experts compiled the *"Chinese Expert Consensus on the Diagnosis and Treatment of Autoimmune Encephalitis"* based on the clinical characteristics of AE in China, pointing out that this new type of AE is a treatable (by detecting pathogenic antibodies and diagnostic markers) and not uncommon encephalitis, and that the prognosis for the patient who has received immunotherapy in early stage and suffered from a non-severe condition will be better. The diagnosis of AE first requires a comprehensive analysis of the patient's clinical manifestations, an examination of cerebrospinal fluid, neuroimaging, electroencephalogram and other ancillary detection results to determine that the patient is suffering from encephalitis (suspected AE or possible AE), and subsequent a known AE related antibody test is performed to diagnose. If the antibody test is positive and consistent with the clinical manifestation and other possible causes are excluded, AE can be definitively diagnosed as an AE directed against a certain known autoantigen, such as anti-NMDAR encephalitis. Therefore, the positive result for the detection of the corresponding antibody is a necessary condition for the diagnosis of AE but not a sufficient condition. Since the clinical manifestations of autoimmune encephalitis in children are different from those in adults, it is difficult to identify specific autoimmune encephalitis syndrome. Therefore, the detection of autoantibodies is very important for the definite diagnosis of autoimmune encephalitis in children.

With the establishment of the consensus, the positive result of AE antibody becomes a necessary condition for the diagnosis of AE. Currently, AE-specific antibodies are mainly detected using cell based assay (CBA) based on the expression of specific antigens. In 2014, the Chinese Academy of Medical Sciences conducted anti NMDAR antibody testing on the cerebrospinal fluid of 284 patients with unexplained encephalitis using a single molecule fluorescence CBA method. Among them, 46 of cases were positive for the antibody, with a prevalence rate of 16.2%. This indicates that anti-NMDAR encephalitis is not rare in China. Therefore, it is necessary to conduct anti-NMDAR antibody testing for cases of encephalitis of unknown etiology. In 2017, six most common AE antibodies (six autoantibodies) of 750 suspected AE patients were analyzed by the Research Center of the Pennsylvania State University, in US using the single molecule fluorescence CBA method, and among the detected four AE antibodies, the proportion of NMDAR antibody was the highest, accounting for 10.94%. We tested approximately 4000 suspected AE samples and the total positive detection rate of AE antibody is known to be less than 20%. Early diagnosis of AE remains a challenge. The serum or cerebrospinal fluid from some patients with negative detection of known AE antibodies showed positive results through immunohistochemical examination of mouse brain tissue, indicating that there may be unknown antibodies, which accounted for about 60-80% of AE. Therefore, the discovery of new AE-associated antibodies and their targeted molecular markers, and the further elucidation of their pathogenicity and pathogenesis are of great significance for better clinical diagnosis and treatment of autoimmune encephalitis.

The pathogenesis of AE is still in the initial stage of research, and the occurrence and clinical manifestations of the disease triggered by different autoantibodies may vary. For example, after the anti-NMDAR antibody binds to neuron NMDAR, the number of synapses is greatly reduced; the anti-GABABR antibody inhibits the activity of such receptor, but has no significant effect on the density of synapses; anti-LGI1 and CASPR2 antibodies can interfere with synaptic protein-protein interaction; anti-neurexin-3α antibody can alter the formation of synapses; and anti-IgLON5 antibody can lead to the formation of new forms of Tau protein lesions. Clinically, most patients exhibit symptoms of encephalitis, and some of them also exhibit symptoms of cerebellar syndrome or chronic encephalopathy.

Tumors and viral infections are risk factors for AE. Anti-NMDAR encephalitis is often accompanied by teratoma, and anti-GABABR encephalitis is often accompanied by small cell lung cancer. Tumor cells may secrete autoantigens and thus activate the immune system, leading to the development of AE. For example, neuron-like tissue cells in teratomas secrete NMDAR, and the inflammatory response of tumor tissue in patients with AE is higher than that in those without AE. However, the exception is encephalitis with DNER, mGluR1, and mGluR5 accompanied by Hodgkin lymphoma, where the tumor does not produce any AE-associated antigens. About 20% of patients with herpes simplex virus encephalitis may develop secondary AE (mainly NMDAR) after a few weeks. The mechanism may be that virus-mediated intracranial inflammatory reaction promotes generation of autoantigens, leading to an autoimmune reaction outside the nervous system. However, LG1 encephalitis and many AEs, especially those in young patients, have not been found with tumors or evidence of early viral infection during long-term follow-up, indicating that the mechanism is mostly unclear. The mechanism of AEs still needs to be further investigation.

### SUMMARY

The object of the present disclosure is to provide a novel antibody marker and establish a detection system thereof for diagnosis and treatment of an autoimmune disease of nervous system.

The technical solution adopted by the present disclosure is as follows:
The present disclosure provides use of CRMP2 as a marker for an autoimmune disease of nervous system.

In some embodiments of the present disclosure, the CRMP2 comprises an amino acid sequence as shown in any one of SEQ ID NOs: 1 to 3.

In some embodiments of the present disclosure, the CRMP2 comprises a nucleotide sequence as shown in any one of SEQ ID NOs: 4 to 6.

The present disclosure also provides use of anti-CRMP2 antibody as a marker for an autoimmune disease of nervous system.

The present disclosure also provides use of a reagent detecting anti-CRMP2 antibody in the preparation of a reagent for detection, diagnosis, treatment or prognosis evaluation of an autoimmune disease of nervous system.

In some embodiments of the present disclosure, a method for detecting the anti-CRMP2 antibody is at least one of CBA, immunoblotting, membrane strip method, ELISA, chemiluminescence, radioimmunoassay, liquid chip method, lateral chromatography, electrochemistry, and flow cytometry.

The present disclosure also provides use of a reagent inhibiting expression or translation of anti-CRMP2 antibody in preparation of a medicine for an autoimmune disease of a nervous system.

In some embodiments of the present disclosure, a preparation inhibiting expression or translation of the anti-CRMP2 antibody comprises a gene promoter inhibitor of the anti-CRMP2 antibody, a gene transcription inhibitor of the anti-CRMP2 antibody, a protein synthesis inhibitor of the anti CRMP2 antibody, or a siRNA of the anti CRMP2 antibody gene.

The present disclosure also provides use of a preparation reducing or inactivating an activity of an anti-CRMP2 antibody in preparation of a medicine for an autoimmune disease of nervous system.

In some embodiments of the present disclosure, a reagent reducing or inactivating activity of the anti-CRMP2 antibody comprises a substance for inhibiting the activity of the anti-CRMP2 antibody, a substance for degrading the activity of the anti-CRMP2 antibody, or a gene tool for reducing the protein level of the anti-CRMP2 antibody.

The present disclosure also provides an auxiliary diagnostic reagent for an autoimmune disease of nervous system, the reagent contains a reagent for detecting the expression amount of an anti-CRMP2 antibody.

In some embodiments of the present disclosure, a detected sample is cerebrospinal fluid or blood.

In some embodiments of the present disclosure, the autoimmune disease of the nervous system is encephalitis or encephalomyelitis.

The beneficial effects of the present disclosure are that:
The present disclosure discovers that the CRMP2 is a novel encephalitis or encephalomyelitis-associated autoantibody-targeted molecular marker through research. According to the nomenclature rules of this type of disease, this novel disease is called anti-CRMP2 encephalitis or encephalomyelitis, and the affected area or symptom location may be brain, cerebellum and/or spinal cord. CRMP2 can be used for definitive diagnosis of the disease and provide basis for immunotherapy regimens, and can monitor therapeutic effect and determine prognosis and recurrence by detecting the change in the titer of the autoantibody.

### BRIEF DESCRIPTION OF THE DRAWINGS

FIG. 1 shows a map of pcDNA3.1+ vector.
FIG. 2 shows the images of patients P1, P2 and P3.
FIG. 3 shows the immunohistochemical detection of brain slice from cerebrospinal fluid of patient P1.
FIG. 4 shows the identification of the CRMP2 marker.
FIG. 5 shows an immunofluorescence detection of patient P1 sera from primary cultured mouse cerebral cortical neurons.
FIG. 6 shows the CBA detection of the serum from patients P1 and P3.
FIG. 7 shows the CBA detection of anti-CRMP 2 antibodies in serum samples from patient P2 before and after treatment.
FIG. 8 shows the detection of the membrane strip method for serum sample from patient P2.
FIG. 9 shows the sequence alignment result of 3 isoforms of CRMP2.
FIG. 10 shows the sequence alignment of CRMP2 protein from human, mouse, rat, cow, orangutan, and chicken.

### DETAILED DESCRIPTION

The concept of the present disclosure and the technical effects thereof will be clearly and completely described below in connection with the embodiments, so as to fully understand the objectives, features and effects of the present disclosure. Obviously, the described embodiments are merely a portion of the embodiments of the present disclosure, rather than all of embodiments. Based on the embodiments of the present disclosure, other embodiments obtained by those skilled in the art without creative labor are also within the scope of protection of the present disclosure.

Hereof, CRMP2 is known to have 3 protein isoforms, and the sequences are as follows:
Isoform 1: length: 677 amino acids; species: human (Homo sapiens), with the amino acid sequence as shown in SEQ ID NO: 1; isoform 2: length: 572 amino acids; species: human (Homo sapiens), with the amino acid sequence as shown in SEQ ID NO: 2; isoform 3: length: 536 amino acids; species: human (Homo sapiens), and with the amino acid sequence as shown in SEQ ID NO: 3.

CRMP2 is known to have 3 transcripts encoding proteins corresponding to the above 3 protein isoforms, and the sequences are as follows:
Transcript 1: length: 2034 bp; species: human (Homo sapiens), with the nucleotide sequence as shown in SEQ ID NO: 4; transcript 2: length: 1719 bp; species: human (Homo sapiens), with the nucleotide sequence as shown in SEQ ID NO: 5; transcript 3: length: 1611bp; species: human (Homo sapiens), with the nucleotide sequence as shown in SEQ ID NO: 6.

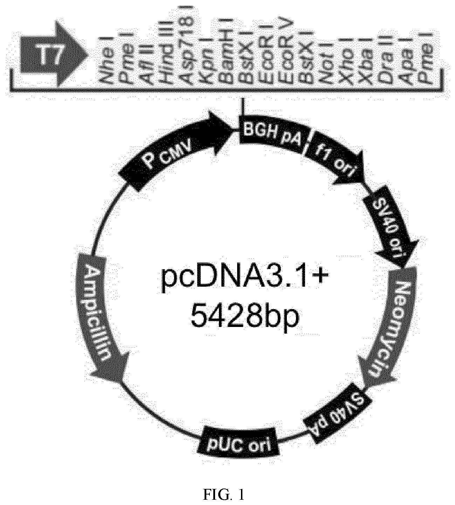

The CRMP2 sequence used in the pcDNA3.1-CRMP2 plasmid is transcript 1 for expression of CRMP2 protein type 1, and covers all the sequences of 3 transcripts and 3 isoforms in order to detect anti-CRMP2 antibodies without omission. Of these, the map of the pcDNA3.1-CRMP2 vector is shown in FIG. 1.

The eukaryotic expression vector used for pcDNA3.1-CRMP2 plasmid is pcDNA3.1+, and the map is as follows: the enzyme cleavage sites used for vector construction are BamH I (5' end) and EcoR I (3' end), with the inserted CRMP2 transcript 1 sequence in the middle. The specific sequence is as shown in SEQ ID NO: 7.

### Example 1

The three patients in this Example were numbered P1, P2 and P3, respectively.

P1 was the first case in which CRMP 2 was identified using cerebrospinal fluid and blood samples therefrom, and P2 and P3 were positive cases of anti-CRMP2 antibody detected by retrospective analysis using CBA method.

P1, P2 and P3 all had symptoms of dizziness or headache, visual impairment and unsteady walking; as well as white matter lesions; with effective immunotherapy, positive TBA detection in mouse brain slices of blood and/or cerebrospinal fluid, positive detection for anti-CRMP2 antibody and negative detection for other AE associated antibodies.

P1 and P3 had speech barriers and inaccuracy in bilateral finger-nose test, and P2 had chest and abdominal pain, and spinal cord lesions.

For P1, cerebrospinal fluid was tested positive for *Mycoplasma pneumoniae,* and for P3, cerebrospinal fluid was tested positive for human herpesvirus type 6 and *Klebsiella pneumoniae,* and the IgM of influenza B virus in blood was positive.

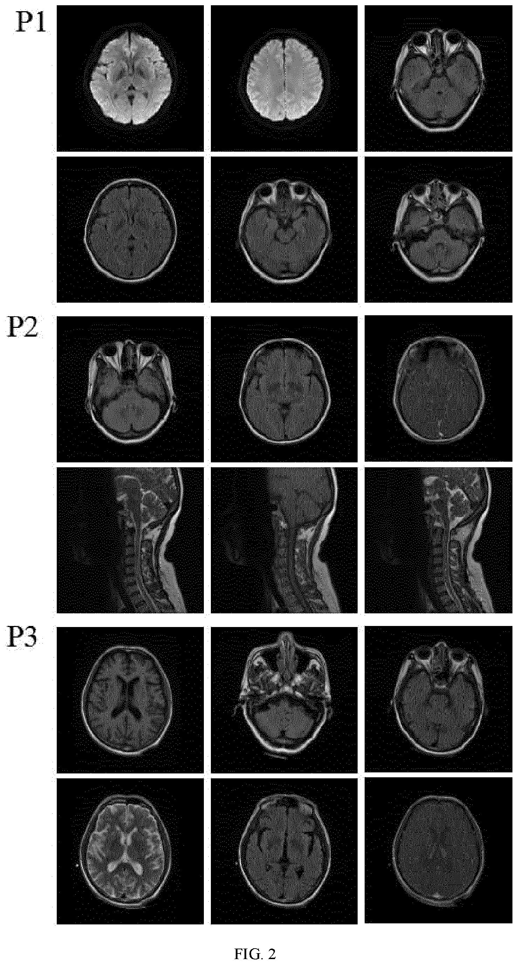

The specific clinical data of these 3 patients, P1, P2 and P3, were shown in Table 1, and the imaging data were shown in FIG. 2.

**Table 1 Clinical data of 3 patients with anti-CRMP2 antibody positive encephalitis/encephalomyelitis**

| Patie nt | Gender | Age (year of age, approximat e age,for protection of privacy) | Symptoms | Physical examination | Cerebrospinal fluid | Brain slice |
|---|---|---|---|---|---|---|
| P1 | Female | About 35 | Dizziness, rotation of vision, unsteady walking, and fever | Poetry-like speech, normal muscle strength of limbs, poor stability and inaccuracy in bilateral finger-nose test, poor coordination movement and slight resistance of neck | Leukocyte 38/µL, mononuclear cell 90% | Brain section and cerebellu m section: cerebrosp inal fluid (+) |
| P2 | Female | About 40 | Headache, nausea, emesis, chest pain on the left side, weakness in the right upper limb, followed by weakness in both lower limbs. | Decreased binocular vision; limb muscle strength at grade 4; Romberg (+), unsteady walking in a straight line, and poor coordinate movement; sensitivity to pain in the left chest and abdomen, decreased acupuncture sensation in the left back, and abnormal sensation in both feet. | Pressure 185 mmH₂O, Leukocyte 240/µL, erythrocyte 20/µL, protein 0.80g/L and sugar 2.64mmol/L. | Brain section and spinal cord section: serum (+), cerebrosp inal fluid (-) |
| P3 | Male | About 65 | Dizziness, unstable standing, diplopia, emesis, glossolalia, choking when drinking. | Vague speech, free movement of both eyeballs in all directions, and visible nystagmus; limb muscle strength at grade 4, and poor vibration perception of tuning forks in both ankles, and normal feelings on depth of the whole body; inaccuracy in bilateral finger-nose test and heel-knee-tibi a test. Romberg, fine motor skills disorder, unskillful button-up, and agraphia | Sugar 4.89 mmol/L, no abnormalities found in biochemical and conventional detections of the cerebrospinal fluid | Brain section and spinal cord section: serum (+), cerebrosp inal fluid (-) |

| Patie nt | Autoimmune antibodv/antigen | Other test indexes | Images | Preliminary diagnosis | Immunotherap y | Prognosis |
|---|---|---|---|---|---|---|
| P1 | Autoimmune encephalitis antibody¹ (cerebrospinal fluid, serum), autoimmune cerebellar-asso ciated antibody² (serum), thyroid-associa ted antibody³, tumor antigen⁴ (-) | Mycoplas ma pneumonia e antibody (+), next generation sequencing of cerebrospin al fluid genes: 99% of mycoplasm a | MRI: mild leukoencephalo pathy | Encephalitis (Mycoplasma) | Immunoglobul in, methylprednis olone; | Mild dizziness and opsoclon us on discharge; 0 mRS score at the 3rd month |
| P2 | Anti-GFAP antibody (cerebrospinal fluid) (-), anti-MOG antibody, anti-AQP4 antibody (cerebrospinal fluid, serum) (-), thyroid associated antibody (-), anti-histone antibody (+), and the rest of antiserum autoimmune antibody⁵ (-) | Mycobacte rium tuberculosi s γ -interferon release test (-), mycobacter ium tuberculosi s DNA (-), x-pert (-) and no acid-fast bacilli detected in acid-fast microscopy. | MRI: visible long-segment lesion of spinal cord, multiple flaky abnormal signals of white matter regions of bilateral cerebral hemispheres and brainstem, and considering demyelinating lesion | 1. Acute disseminated encephalomy elitis; 2. tuberculous meningitis? | Methylprednis olone | Symptom s improved on discharge, and 1 mRS score at the 3rd month |
| P3 | 12 antibodies against paraneoplastic neurological syndrome in serum were all negative, including anti-GAD65, ITPR1, CRMP5, Zic4, Tr (DNER), Homer3 and ATP1A3 antibodies (-), anti-AQP4, NMDA, MOG and GFAP receptor antibodies (-), serum autoimmune antibodies (-) and immunofixatio n electrophoresis (-). | Cerebrospi nal fluid pathogen nucleic acid:human herpesvirus type 6, *Klebsiella pneumonia e* (+); IgM of influenza B virus in blood (+) | MRI: 1. Multiple non-acute lacunar cerebral infarction occurred in bilateral basal ganglia and the right side of brain stem, and some softening lesions were formed; 2. mild white matter degeneration; mild brain atrophy; 3. partially the empty sella | Multiple system atrophy (MSA-type C) | Immunoglobul in, methylprednis olone | Dizziness and weakness still existed on discharge, and the dysarthria improved slightly. |

| | | | | | | |
|---|---|---|---|---|---|---|
| Notes: 1.^{1.}Autoimmune encephalitis antibody: the antibodies of anti-NMDA/LGI 1/GASPR-2/DABAB/AMPA1/AMPA2/IgLON5/DPPX/GIy1/DRD2/GAD6. ^{2.}autoimmune cerebellar-associated antibodies: antibody of anti-DPYSL 5/Homer3. ^{3.}thyroid-associated antibodies: antibodies of thyrotropin receptor, antibodies of anti-thyroid peroxidase, antibodies of anti-thyroglobulin; ^{4.}tumor antigen: carbohydrate antigen (CA-125), non-small cell lung cancer-associated antigen, squamous cell carcinoma-associated antigen (SCC), serum pancreas and gastrointestinal tumor-associated antigen (CA 242), gastric cancer-associated antigen (CA-724), gastrointestinal cancer-associated antigen (CA-199), carcinoembryonic antigen (CEA); ^{5.}serum autoimmune antibodies: antibody of antinuclear, antibody of anti-double-stranded DNA, antibody of anti-Jo-1, antibody of anti-ssB, antibody of anti-Sm, antibody of anti-UI-nRNP, antibody of anti-ribosomal P protein, antibody of anti-proliferative nuclear antigen, antibody of anti-mitochondrial, antibody of anti-centromere, antibody of anti-PM-Scl, antibody of anti-SCL-70, antibody of anti-ssA, antibody of anti-nucleosome, antibody of anti-histone, antibody of anti-recombinant Ro52. | | | | | | |

### 2. Abbreviation: MRI: magnetic resonance; mRS: modified Rankin scale.

### Example 2

Immunohistochemical experiment based on rat brain slices is a routine examination of autoimmune encephalitis, including immunoenzyme chemical method and immunofluorescence (also known as TBA, tissue based assay).

Implementation process: The adult rat was anesthetized and then the heart of adult rat was infused with PBS (phosphate buffer) to remove circulating blood. The brain (including hippocampus) was taken for sagittal frozen sections. The cerebellum was taken to be longitudinally sliced, fixed by cold acetone for 10 minutes and dried for later use. Slices were washed with PBST solution (PBS containing 0.05% of Tween-20) for 3 times, 3 minutes each time.

Next, immunoenzyme chemical and immunofluorescence methods were performed for detection respectively: (1) immunoenzyme chemical method was used: the slices were then treated with 3% hydrogen peroxide for 10 minutes, washed with PBST for 3 times prior to the next step of blocking, and blocked with 10% goat serum/PBST solution for 1 hour at room temperature; (2) immunofluorescence method was used: without hydrogen peroxide treatment, the slices were blocked with 10% goat serum/PBST solution for 1 hour at room temperature.

The blocking buffer was shaken off after the two groups were blocked, and the cerebrospinal fluid from patients P1 was added, and incubated at 37°C for 1 hour. After incubation, the slices were washed with PBST for 3 times, 5 minutes each time.
(1) For immunoenzyme chemical method: after incubation and washing of cerebrospinal fluid, a Horseradish Peroxidase (HRP) labeled goat anti-human IgG secondary antibody (bs-0297G-HRP, Bioss) with a dilution of 1:5000 was added, incubated at 37C for 30 minutes, and then diaminobenzidine (Diaminobenzidine) was added dropwise after washing. The staining lasted for 3-15 minutes, after rinsing, the nucleus was re-stained, sealed with resin, and the images were observed and collected under a DM3000 microscope (Leica, German).
(2) For immunofluorescence method: a green fluorescently labeled goat anti-human IgG secondary antibody (ab 97003, Abcam) with a dilution of 1:200 was added and incubated at 37 °C for 30 minutes. The slices were then washed with PBST for 3 times, 5 minutes each time. Anti-quenching sealing agent was used to seal, and IX73 fluorescence microscopy (Olympus, Japan) was used to observe the results and collect images.

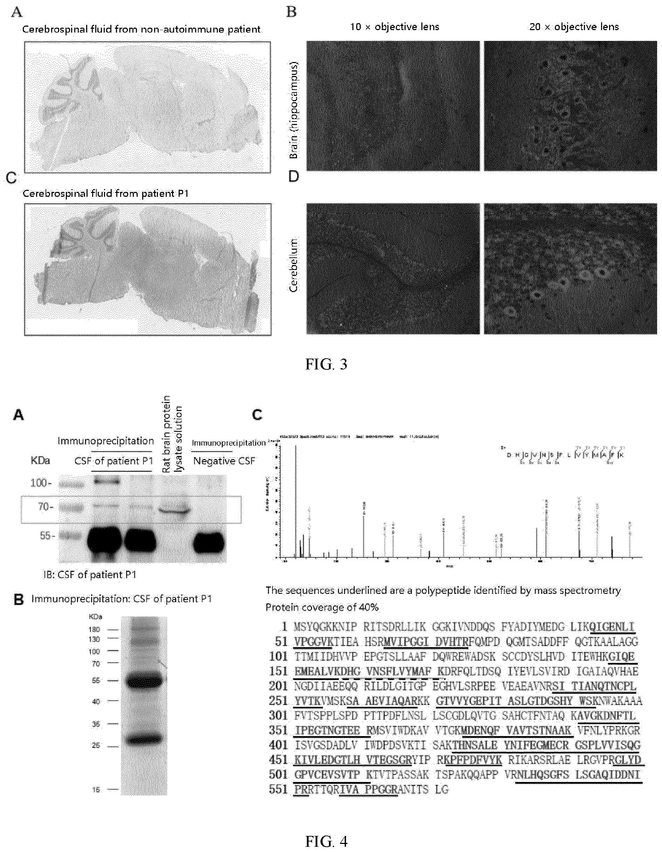

Results: The detection results are shown in FIG. 3, in which Images A and C in FIG. 3 are the results of immunoenzyme chemical detection. Wherein the comparison results of cerebrospinal fluid staining of negative non-autoimmune patients are shown in Image A in FIG. 3, and positive cerebrospinal fluid staining for patient P1 is shown in Image C in FIG. 3. Images B and D in FIG. 3 show the results of immunofluorescence detection, Image B in FIG. 3 shows the results of detection in hippocampus of brain, and Image D in FIG. 3 shows the results of detection in Purkinje cells of cerebellum, all of which are positive. This indicates that the cerebrospinal fluid of patient P1 contained autoantibodies against cerebral neurons, and patient P1 has a suspected AE.

### Example 3

Antigens against autoantibodies in cerebrospinal fluid from patient P1 were identified using immunoprecipitation combined with protein mass spectrometry.

Implementation process: Fresh brain tissue of rats aged about 2 months was taken, 1 mL of a protein lysate agent (20 mmol/L Tris-HCl, pH 7.4, 150 mmol/L NaCl, 2 mmol/L EDTA, 1% Triton X-100, protease inhibitor) was added and homogenized in a glass homogenizer for 1 minute, and then placed on ice to lysis for 30 minutes. Centrifugation was performed at 12000 rpm at 4 °C for 15 minutes, and the supernatant was collected as rat brain protein lysate solution. 500 µL of protein lysate solution was mixed with 500 µL of cerebrospinal fluid from patient P1, and vertically mixed and rotated at 4°C overnight. 50 µL of pre-washed protein A labeled agarose was added, and vertically mixed and rotated for 2 hours at 4 °C. The mixture was centrifuged at 1000 rpm at 4 °C, resuspended, rinsed with protein lysate agent for 3 times, and then added 1 × SDS-PAGE sample buffer to boil for 5 minutes to elute protein complex. The prepared samples were separated by SDS-PAGE for immunoblotting and Coomassie brilliant blue staining, respectively. The cerebrospinal fluid which was tested negative in TBA detection of rat brain slices was used as negative control. Based on the experimental results, the suspected target Coomassie brilliant blue staining bands (about 70KDa) were selected, cut off gel for peptide fingerprint mass spectrometry protein identification. The results are shown in FIG. 4. Of these, Image A in FIG. 4 shows the result of immunoprecipitation. Image B in FIG. 4 shows the result of Coomassie brilliant blue staining, where the immunoprecipitation and Coomassie brilliant blue staining indicates that a target protein band may be at about 70Kd,. After gel cutting and mass spectrometry identification, more than 10 peptides of CRMP2 were identified. Of these, Image C in FIG. 4 shows the secondary mass spectrometry of one of the polypeptides, and image D in FIG. 4 shows the distribution of the polypeptide (underlined) identified by protein mass spectrometry on CRMP2 protein, in which the CRMP2 protein coverage of the polypeptide is 40%. The polypeptide underlined with a dotted line in image D in FIG. 4 corresponds to the polypeptide in image C in FIG. 4.

### Example 4

CRMP2 was located in the cytoplasm, axons and dendrites of neurons. Anti-CRMP2 antibodies in cerebrospinal fluid and serum samples from patients with anti-CRMP2 encephalitis/encephalomyelitis should be able to bind to neurons, which is an important mechanism arising AE.

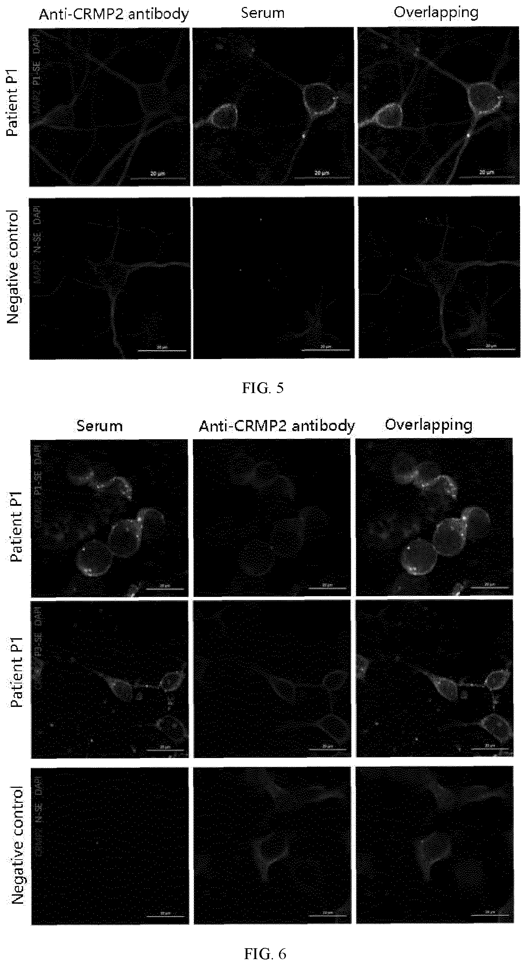

Implementation process: C57 mouse embryos of 18-19 days old were taken, and their brain tissues were separated to prepare cortical neuron cell suspension. A 24-well cell culture plate placed in advance with cell slides was laid at 3 × 10⁶/well and cultured for 14 days. After cultivation, neurons were fixed with icy acetone for 10 min. After washed by PBST 3 times for 3 minutes, and blocked by 10% goat serum/PBST solution at room temperature for 1 hour, the serum from patients or control group (a dilution of 1: 20) and anti-MAP2 antibody (8707T, Cell Signaling Technology, a dilution of 1: 200) were added, and incubated at 4°C overnight or 37°C for 1 hour. After the reaction, washed by PBST 3 times for 5 minutes, and then goat anti-rabbit IgG (ab 150092, Abeam) labeled with red fluorescence (Alexa Fluor 594) and goat anti-human IgG (ab 97003, Abeam) labeled with green fluorescence (DyLight 488) were added and incubated at 37 °C for 30 minutes. Washed by PBST 3 times for 5 minutes, and then sealed with sealing agent containing DAPI (4', 6-diamidino-2-phenylindole, Dihydrochloride). Photographs were taken using a LSM 980 laser confocal microscope (zeiss, German). The results are shown in FIG. 5. The scale is 20 µm.

As can be seen from FIG. 5, the serum stained neurons of patient P1 are positive, and the signals are distributed on the cytoplasm, dendrites and axons of neurons, which were consistent with the localization of CRMP2 and consistent with the characteristics of anti-CRMP2 encephalitis/encephalomyelitis. Negative control sera were not stained.

### Example 5

Detection for cerebrospinal fluid and serum samples of patients by immunofluorescence (i.e., the CBA method) based on 293T cells overexpressing target antigen is an important way to diagnose autoimmune encephalitis/encephalomyelitis.

Implementation process: a 293T cell slide was prepared, and when the cells grew to 50% - 90% of density, the eukaryotic expression plasmid pcDNA3.1-CRMP2 was transfected into 293T cells to make the 293T cells overexpress the CRMP2 recombinant protein. The 293T cells were washed with PBS for 3 times, then fixed by acetone for 5 minutes and dried. The slide was washed with PBST solution for 3 times, 3 minutes each time. Blocked with 10% goat serum/PBST solution for 1 hour at room temperature, the sealing solution was abandoned, and serum from patient P1 or P3 (diluted at 1: 20) was added, and incubated at 37 °C for 1 hour. After incubation, the cell was washed with PBST for 3 times, 5 minutes each time. Then a 1: 200-fold dilution of a green fluorescently labeled goat anti-human IgG secondary antibody (ab 97003, Abeam) was added and incubated at 37 °C for 30 minutes. Washed with PBST for 3 times, 5 minutes each time. The sealing agent containing DAPI was used to seal, and LSM 980 laser confocal microscope (zeiss, German) was used to observe the results and collect images.

In this example, while adding the first antibody, an anti-CRMP2 antibody (ab129082, Abeam, a dilution of 1:200) was added as a positive control, at the same time as the secondary antibody, a red fluorescence-labeled goat anti-rabbit IgG secondary antibody (ab 150092, Abeam) was added to for immunofluorescence co-staining. This example uses serum from patients with non-autoimmune diseases as a negative control. The results are shown in FIG. 6.

As can be seen from FIG. 6, this example successfully overexpressed CRMP2 protein in 293T cells, and the serum staining from patients P1 and P3 substantially overlapped with CRMP2. The negative control serum were not stained. It indicated that the serum from patients P1 and P3 contained autoimmune antibodies against CRMP2.

### Example 6

Reduction or elimination of autoimmune antibodies by immunotherapy is a main way to treat autoimmune encephalitis/encephalomyelitis.

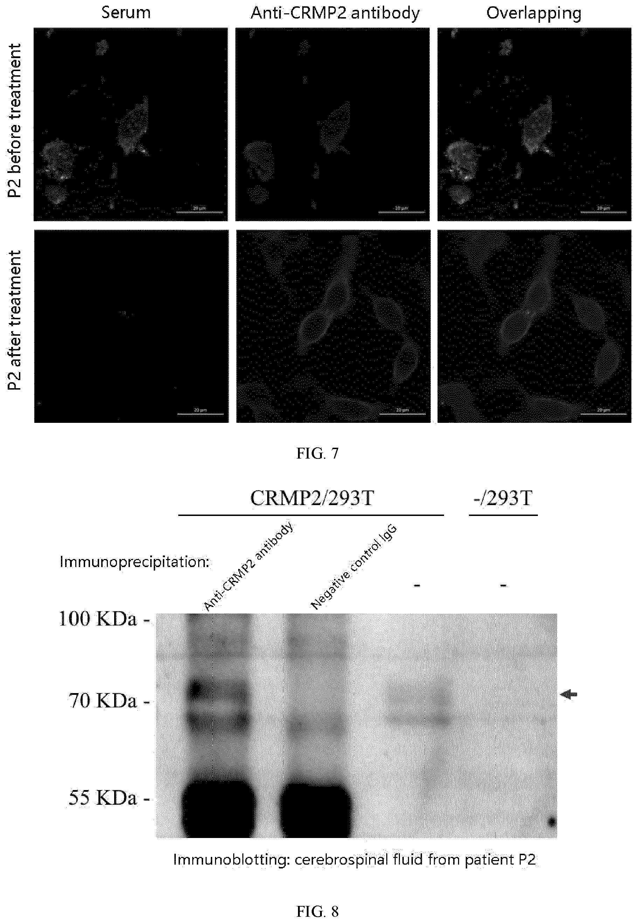

Implementation process: All 3 patients got better after immunotherapy, as shown in Table 1 for immunotherapy and prognosis. A CBA detection for anti-CRMP2 antibodies was performed for serum from patient P2 before and after treatment, with a method same as in Example 5. The results are shown in FIG. 7.

As can be seen in FIG. 7, the serum from patient P2 before treatment was positive for CBA staining and co-localized with CRMP2, which indicates that the serum from patient P2 before treatment contained autoimmune antibodies against CRMP2. After treatment and recovery, the serum from patient P2 was negative for CBA staining, which indicates that the anti-CRMP2 antibodies in the serum disappeared after treatment of patient P2.

### Example 7

Immunoblotting (also known as membrane strip method) is another method for detecting autoimmune antibodies.

Implementation process: First, the CRMP2 protein was prepared. 293T cells were cultured in a 10 cm cell culture dish to a density of 80%, pcDNA3.1-CRMP2 eukaryotic expression plasmids were transfected into the 293T cells using EntransterTM-D4000 (4000-2, Engreen), and the 293T cells were continuously cultured for 24 to 48 hours for overexpression of CRMP2 recombinant proteins. Then, the 293T cells were treated with 1 mL of protein lysate agent at 4 °C for 30 min. Centrifugation was performed at 12000 rpm at 4°C for 15 min and the supernatant was collected to obtain CRMP2-containing 293T protein lysate solution. 5 µg of anti-CRMP2 antibody (ab129082, abeam) was added, and vertically mixed and rotated at 4 °C overnight. 50 µL of pre-washed protein A labeled agarose was added, and vertically mixed and rotated for 2 hours at 4 °C. Centrifugation was performed at 1000 rpm at 4°C to remove supernatant, and resuspended with protein lysate agent, followed by repeated centrifugation and resuspension to rinse 3 times. After the last centrifugation, 30 µL of 1 × SDS-PAGE sample buffer was added for resuspension, and boiled for 5 min to elute, purify and enrich CRMP2 protein. The eluent was separated by 10% SDS-PAGE, transferred to a nitrocellulose membrane, blocked with 5% skim milk powder/TBST at room temperature for 1 hour, incubated with 1:20 diluted cerebrospinal fluid from P2 patient at room temperature for 2 hours, washed 3 times for 5 min by TBST, incubated with 1:5000 diluted HRP-labeled goat anti-human IgG at room temperature for 1 hour, washed 3 times for 5 min by TBST, and developed with ECL substrate for detection of the anti-CRMP2 antibodies in the cerebrospinal fluid of the P2 patient. Normal rabbit IgG was used as a negative control for anti-CRMP2 antibody,. The pcDNA3.1 empty vector transfected 293T was used as a negative control for the CRMP2 protein. The results are shown in FIG. 8. As can be seen from FIG. 8, a clear band appeared at the position of the target CRMP2 size, and the corresponding negative control had no band, which indicates that the cerebrospinal fluid of the P2 patient contained anti-CRMP2 antibody. At the same time, it indicates that immunoblotting (membrane strip method) can be used for the detection of such antibody.

### Example 8

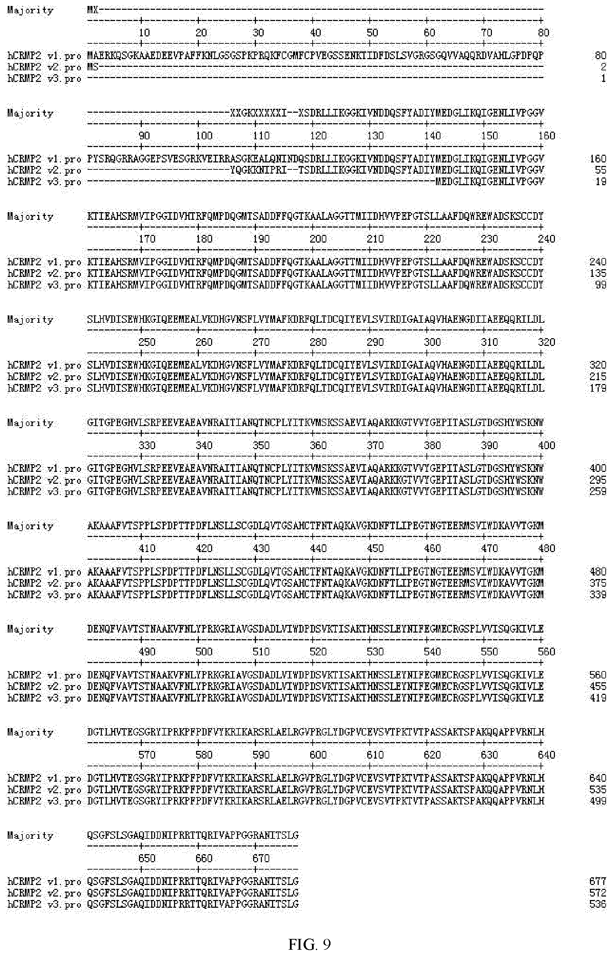

The results of sequence alignment of the 3 isoforms of CRMP2 are shown in FIG. 9, which shows that the amino acid sequences of the 3 isoforms are completely consistent at the positions 142 to 677. Therefore, the CRMP2 sequence in the pcDNA3.1-CRMP2 plasmid used in Examples 5, 6 and 7 is transcript 1 for expression of CRMP2 protein type 1, and covers all the sequences of 3 transcripts and 3 isoforms in order to detect anti-CRMP2 antibodies without omission.

### Example 9

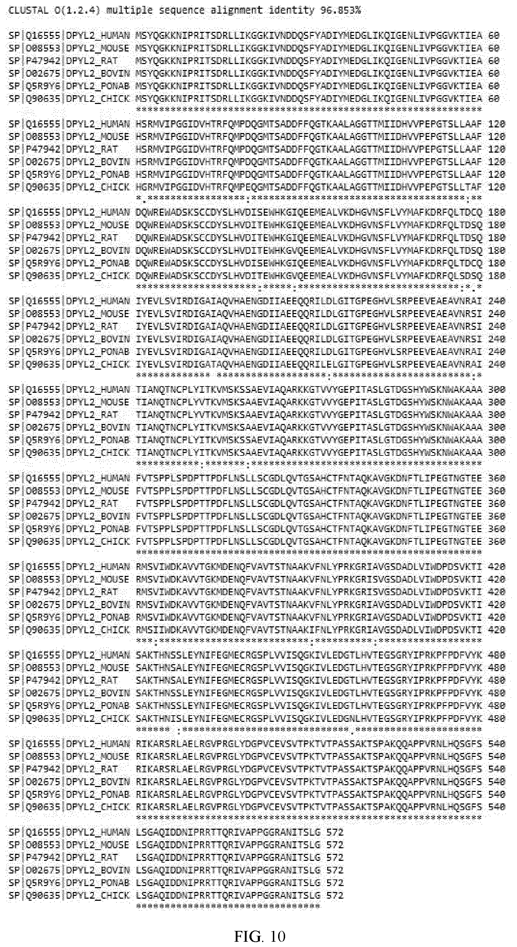

Homology analysis was performed on the protein sequence of CRMP2 (also known as DPYL2) from different species, and the results are shown in FIG. 10. It can be seen from FIG. 10 that CRMP2 proteins of different species are highly homologous, with a homology of 96.853%. The CRMP2 proteins from different species can be used to detect anti-CRMP 2 antibodies in human cerebrospinal fluid or serum samples.

The above specific embodiments provide a detailed description for the present disclosure, but the present disclosure is not limited to the above embodiments, and various changes can be made within the scope of knowledge of those skilled in the art without departing from the objectives of the present disclosure, Furthermore, embodiments of the present disclosure and features in these embodiments may be combined with each other without conflict.

## Claims

1. Use of CRMP2 as a marker for an autoimmune disease of nervous system.

2. Use of anti-CRMP2 antibody as a marker for an autoimmune disease of nervous system.

3. Use of a reagent detecting anti-CRMP2 antibody in the preparation of a reagent for detection, diagnosis, treatment or prognosis evaluation of an autoimmune disease of nervous system.

4. The use according to claim 3, wherein a method for detecting the anti-CRMP2 antibody is at least one of CBA, immunoblotting, membrane strip method, ELISA, chemiluminescence, radioimmunoassay, liquid chip method, lateral chromatography, electrochemistry, and flow cytometry.

5. Use of a reagent inhibiting expression or translation of anti-CRMP2 antibody in the preparation of a medicine for an autoimmune disease of nervous system.

6. The use according to claim 5, wherein the reagent inhibiting the expression or translation of the anti-CRMP2 antibody comprises a gene promoter inhibitor of the anti-CRMP2 antibody, a gene transcription inhibitor of the anti-CRMP2 antibody, a protein synthesis inhibitor of the anti CRMP2 antibody, or a siRNA of an anti CRMP2 antibody gene.

7. Use of a reagent reducing or inactivating an activity of an anti-CRMP2 antibody in the preparation of a medicine for an autoimmune disease of nervous system.

8. The use according to claim 7, wherein the reagent reducing or inactivating the activity of the anti-CRMP2 antibody comprises a substance for inhibiting the activity of the anti-CRMP2 antibody, a substance for degrading the activity of the anti-CRMP2 antibody, or a gene tool for reducing a protein level of the anti-CRMP2 antibody.

9. An auxiliary diagnostic reagent for an autoimmune disease of nervous system, wherein the reagent comprises a reagent for detecting a level of anti-CRMP2 antibody.

10. The use according to any one of claims 1 to 8 or the reagent according to claim 9, wherein the autoimmune disease of the nervous system is encephalitis or encephalomyelitis.
